# EUROPÄISCHE PATENTSCHRIFT

(11) **EP 2 063 939 B1**
(45) Veröffentlichungstag und Bekanntmachung des Hinweises auf die Patenterteilung: **19.01.2011**
(21) Anmeldenummer: 07800629.3
(22) Anmeldetag: 05.09.2007
(51) Int. Cl.: A61M 5/32

(54) **NADELSCHUTZVORRICHTUNG MIT BLOCKIERTER SCHUTZPOSITION**
NEEDLE PROTECTION DEVICE WITH A BLOCKED PROTECTION POSITION
DISPOSITIF DE PROTECTION D'AIGUILLE À POSITION DE PROTECTION BLOQUÉE

(30) Priorität: 06.09.2006 DE 202006014719 U; 06.09.2006 DE 202006014720 U; 10.11.2006 DE 102006053055
(43) Veröffentlichungstag der Anmeldung: 03.06.2009
(73) Patentinhaber: Tecpharma Licensing AG, 3401 Burgdorf (CH)
(72) Erfinder: GRATWOHL, Christian, 5000 Aarau (CH); WYMANN, Martin, 3097 Liebefeld (CH); LANZ, Marc, 3268 Lobsigen (CH); WIDMER, Urs, CH-3013 Bern (CH)
(86) Internationale Anmeldenummer: PCT/CH2007/000438
(87) Internationale Veröffentlichungsnummer: WO 2008/028312

(56) Entgegenhaltungen:
- EP-A- 1 557 191
- WO-A-01/91837
- US-A- 4 894 055
- US-A1- 2003 014 018
- US-A1- 2005 277 893

## Beschreibung

Die Erfindung betrifft eine Nadelschutzvorrichtung, die an einem Injektionsgerät befestigt oder befestigbar ist. Die Nadelschutzvorrichtung findet bevorzugt in der Verabreichung, beispielsweise Selbstverabreichung von Medikamenten Verwendung, beispielsweise der Verabreichung von Insulin, und kann insbesondere in der Selbstverabreichung verwendet werden, d.h. von Patienten, die sich das betreffende Medikament selbst verabreichen. Das Injektionsgerät kann eine einfache Spritze sein, auch eine Spritze, die nach einmaligem Gebrauch entsorgt wird. In derartigen Verwendungen kann die Nadelschutzvorrichtung entweder separat von dem Injektionsgerät hergestellt und für die Verabreichung mit dem Injektionsgerät verbunden werden oder gleich integrierter Bestandteil des Injektionsgeräts sein und gemeinsam mit diesem entsorgt werden. Bevorzugter handelt es sich bei dem Injektionsgerät jedoch um eines für wiederholten Gebrauch, und besonders bevorzugt handelt es sich um ein Injektionsgerät, das die Einstellung der Dosis des zu verabreichenden Produkts zulässt. Das Injektionsgerät kann insbesondere ein Injektionspen sein, wie er beispielsweise in der Diabetestherapie und mittlerweile auch anderen Therapien verwendet wird.

Bei der Handhabung von Injektionsgeräten besteht die Gefahr, dass Patienten oder medizinisches Fachpersonal sich an spitzen Injektionsnadeln verletzen und an einer bereits einmal benutzten Injektionsnadel infizieren. Um dies zu verhindern, wurden Nadelschutzvorrichtungen mit beweglichem Nadelschutz entwickelt. Für die Injektion wird das mit der Nadelschutzvorrichtung ausgestattete Injektionsgerät mit dem Nadelschutz gegen die Haut gedrückt. Das Injektionsgerät mit der davon in die distale Richtung vorragenden Injektionsnadel bewegt sich aufgrund der äußeren Kraft relativ zu dem Nadelschutz in die distale Richtung, so dass die Injektionsnadel die Haut durchsticht. Der Nadelschutz vollführt relativ zu dem Injektionsgerät entsprechend eine Bewegung in die proximale Richtung bis in eine Freigabeposition, in der die Injektionsnadel mit ihrem in die Haut eindringenden Injektionsabschnitt in distaler Richtung über den Nadelschutz vorsteht. Wird die Injektionsnadel wieder aus der Haut gezogen, bewegt sich der Nadelschutz aufgrund der Beaufschlagung mit einer Elastizitätskraft eines Federglieds wieder in die distale Richtung, bis in eine Schutzposition, in der er die Injektionsnadel einschließlich einer Nadelspitze überragt. Mit Erreichen der Schutzposition verriegelt sich der Nadelschutz selbsttätig, so dass er sich relativ zu der Injektionsnadel nicht wieder in die proximale Richtung zurückbewegen kann.

Aus der US 6,773,415 B2 und der WO 01/91837 A1 sind derartige Nadelschutzvorrichtungen bekannt. Der Nadelschutz gelangt jeweils am Ende seiner Einfahrbewegung in einen irreversiblen Eingriff, der bewirkt, dass sich der Nadelschutz in die distale Richtung nicht mehr in die Ausgangsposition, sondern nur noch in die blockierte Schutzposition bewegen kann, wenn er sich selbst überlassen, d. h. von der äußeren Kraft entlastet wird.

Es ist eine Aufgabe der Erfindung, eine Nadelschutzvorrichtung zu schaffen, die einfach herstellbar ist und den Verwender noch sicherer vor Kontakt mit der Injektionsnadel schützt.

Die Erfindung geht von einer Nadelschutzvorrichtung aus, die an einem Injektionsgerät befestigt oder für eine Befestigung vorgesehen oder fester Bestandteil des Injektionsgeräts ist. Sie umfasst einen Nadelhalter mit einer in eine distale Richtung vorragenden Injektionsnadel und ein Federglied, das den Nadelschutz direkt oder gegebenenfalls erst über ein einziges oder mehrere Zwischenglied(er) in die distale Richtung mit einer Federkraft beaufschlagt. Die Nadelschutzvorrichtung umfasst ferner eine Blockiereinrichtung, die vorzugsweise ebenfalls in die distale Richtung über den Nadelhalter vorragt. Die Blockiereinrichtung ist oder umfasst vorzugsweise ein Hohlprofil, das die Injektionsnadel über einen Längenabschnitt umlaufend umgibt. Bestandteil der Nadelschutzvorrichtung ist ferner ein Nadelschutz, der aus der distalen Ausgangsposition in die proximale Richtung bis in eine die Injektionsnadel freigebende Freigabeposition und aus einer Freigabeposition in die distale Richtung bis in eine Schutzposition bewegbar ist, in der er die Injektionsnadel einschließlich einer Nadelspitze umgibt. Die Blockiereinrichtung und der Nadelschutz wirken auf eine Weise zusammen, die sicherstellt, dass der Nadelschutz in der Schutzposition gegen eine erneute Bewegung in die proximale Richtung blockierbar ist, d.h. bei Erreichen der Schutzposition in dieser blockiert wird. Der Nadelschutz umgibt die Injektionsnadel, so dass in der Schutzposition ein Zugriff auf die Injektionsnadel sicher verhindert wird. Bevorzugt ist der Nadelschutz eine umlaufend zumindest im Wesentlichen geschlossene Hülse. Grundsätzlich kann er jedoch auch größere Durchbrechungen aufweisen einschließlich der Möglichkeit, dass er nur aus einem einzigen oder mehreren nach distal vorragenden Fingern besteht, solange er in der Schutzposition nur seine Schutzfunktion erfüllt, d.h. den Verwender vor Stichverletzungen schützt.

Nach der Erfindung ist die Nadelschutzvorrichtung "frühauslösend". Dies bedeutet, dass die Blockiereinrichtung den Nadelschutz nicht erst dann in der Schutzposition blockiert, wenn der Nadelschutz zuvor die Freigabeposition erreicht hat, in der die Injektionsnadel mit ihrer vollen Einstechlänge über den Nadelschutz vorsteht, sondern auch in Fällen, in denen der Nadelschutz nur einen kleineren Teil seines aus der Ausgangsposition in die Freigabeposition führenden Freigabehubs ausgeführt hat und dann sich selbst überlassen, d. h. von der in Richtung auf die Freigabeposition wirkenden äußeren Kraft entlastet wird. Er muss lediglich bis in eine zwischen der Ausgangsposition und der Freigabeposition gelegenen Auslöseposition bewegt werden, um sicherzustellen, dass er sich bei Entlastung nicht wieder in die nichtblockierte Ausgangsposition zurückbewegen kann. Durch die Frühauslösung werden Kontaminationen mit verschmutzten Injektionsnadeln auch in Fällen verhindert, in denen der Nadelschutz bei einer Injektion nicht gänzlich in die Freigabeposition bewegt oder die Injektionsnadel aus anderen Gründen, beispielsweise bei unachtsamer Handhabung, nur mit einem Teil ihrer der Freigabeposition des Nadelschutzes entsprechendem Nadellänge freigelegt wurde.

Die Auslöseposition ist vorzugsweise so gewählt, dass der Nadelschutz höchstens die Hälfte seines Freigabehubs zurückgelegt hat, wenn er die Auslöseposition erreicht. Bevorzugter liegt die Auslöseposition um höchstens ein Drittel des Freigabehubs von der Ausgangsposition des Nadelschutzes entfernt. Der Hub, den der Nadelschutz aus der Ausgangsposition in die Auslöseposition ausführt, ist in bevorzugten Ausführungen so kurz, dass die Spitze der Injektionsnadel über den Nadelschutz noch nicht vorsteht, wenn der Nadelschutz seine Auslöseposition einnimmt.

Der Nadelschutz kann unmittelbar mit der Blockiereinrichtung in einem Eingriff sein, vorzugsweise in einem Führungseingriff, der verhindert, dass der Nadelschutz sich nach Erreichen der Auslöseposition wieder zurück in die nicht blockierte Ausgangsposition bewegen kann, sondern in den Blockiereingriff gelangt und somit seine Schutzposition einnimmt. Beispielsweise kann eines aus Nadelschutz und Blockiereinrichtung ein Eingriffselement und das andere eine Kurvenführung bilden, die das Eingriffselement in einem Führungseingriff führt, wenn sich der Nadelschutz aus der Ausgangsposition in die Freigabeposition bewegt. Die Führung weist einen durchgehenden Hauptzweig auf, längs dem das Eingriffselement geführt wird, bis der Nadelschutz die Freigabeposition erreicht hat, und einen davon abzweigenden Nebenzweig, in den der Nadelschutz bei der Bewegung zurück in die distale Richtung gelangt, wenn er zuvor wenigstens die Auslöseposition erreicht hat, d.h. der Nebenzweig zweigt bei der Auslöseposition von dem Hauptzweig ab.

In bevorzugten Ausführungen umfasst die Nadelschutzvorrichtung jedoch zusätzlich ein Sicherungsglied, das relativ zu wenigstens einem aus Nadelhalter und Blockiereinrichtung beweglich ist, um zum einen die Bewegung des Nadelschutzes in die Freigabeposition zu ermöglichen, aber zum anderen eine Bewegung zurück in die Ausgangsposition zu verhindern, wenn der Nadelschutz zuvor bereits die Auslöseposition erreicht hat. Das Sicherungsglied ist relativ zu dem Nadelhalter oder der Blockiereinrichtung, vorzugsweise relativ zu dem Nadelhalter und der Blockiereinrichtung, aus einer ersten Position in eine zweite Position bewegbar. Es nimmt die erste Position dann ein, wenn sich der Nadelschutz in seiner Ausgangsposition befindet. Die zweite Position nimmt das Sicherungsglied ein, wenn der Nadelschutz aus der Ausgangsposition heraus die Auslöseposition erreicht hat. In einer bevorzugten ersten Ausführung verhindert das Sicherungsglied eine Bewegung des Nadelschutzes in die Schutzposition, solange der Nadelschutz aus seiner Ausgangsposition noch nicht in die Auslöseposition bewegt worden ist. In der zweiten Position lässt es in derartigen Ausführungen hingegen die Bewegung in den Blockiereingriff, d.h. in die Schutzposition zu. In einer bevorzugten zweiten Ausführung verhindert es unmittelbar selbst oder trägt zumindest dazu bei, dass der Blockiereingriff des Nadelschutzes in der Schutzposition gelöst und infolgedessen der Nadelschutz aus der Schutzposition wieder in Richtung auf die Freigabeposition bewegt werden kann. Ferner ist es auch möglich, dass das Sicherungsglied in der zweiten Position beide genannten Funktionen erfüllt, also die Bewegung in die Schutzposition zulässt, wobei diese Bewegung relativ zum Sicherungsglied stattfinden kann, und selbst am Blockiereingriff beteiligt ist.

In der ersten Ausführung können der Nadelschutz ein Eingriffselement und das Sicherungsglied ein Eingriffsgegenelement aufweisen, wobei das Eingriffselement in der Ausgangsposition des Nadelschutzes in die distale Richtung in einem Anschlagkontakt mit dem Eingriffsgegenelement ist. Ferner weist das Sicherungsglied in Querrichtung zu der Längsrichtung der Injektionsnadel neben dem Eingriffsgegenelement einen in Längsrichtung der Injektionsnadel erstreckten Durchgang auf. Alternativ kann der Nadelschutz den Durchgang neben dem Eingriffselement aufweisen. Der Nadelschutz ist mit dem Sicherungsglied so gekoppelt, dass die Bewegung des Nadelschutzes in die Auslöseposition eine Relativbewegung zwischen dem Nadelschutz und dem Sicherungsglied in die Querrichtung bewirkt. Durch diese Querbewegung gelangen das Eingriffselement und das Eingriffsgegenelement aus ihrer Überlappung und somit aus dem Anschlagkontakt, und eines dieser Elemente gelangt in die Überlappung mit dem Durchgang. Vorzugsweise ist der Durchgang an dem Sicherungsglied vorgesehen, so dass das Eingriffselement des Nadelschutzes mit dem Durchgang in die axiale Überlappung gelangt und der Nadelschutz sich dann in seine Schutzposition bewegen kann. Je nachdem, ob der Durchgang an den Nadelschutz oder vorzugsweise an dem Sicherungsglied gebildet ist, kann zwischen dem Durchgang und dem Eingriffselement oder Eingriffsgegenelement vorteilhafterweise ein in die proximale Richtung erstrecktes Trennelement angeordnet sein, beispielsweise in einem Stück mit dem Sicherungsglied oder dem Nadelschutz geformt sein. Die in Längsrichtung der Injektionsnadel gemessene Länge des Trennelements entspricht dem Hub, den der Nadelschutz bei der Bewegung aus der Ausgangsposition bis in die Auslöseposition zurücklegt. Die axiale Länge des Trennelements bestimmt somit die Länge des Hubs bis in die Auslöseposition. Das Trennelement verhindert während dieses Hubs im Eingriff mit vorzugsweise dem Eingriffselement des Nadelschutzes, das die genannte Relativbewegung in Querrichtung stattfinden kann, bevor die Auslöseposition erreicht ist.

Die Nadelschutzvorrichtung weist in einer Weiterbildung eine Kurvenführung für das Sicherungsglied auf. Die Kurvenführung besteht wenigstens aus einer relativ zu der Längsrichtung der Injektionsnadel geneigten Führung und wenigstens einem Eingriffsglied, das in einem Führungseingriff längs der Führung geführt wird. Die Neigung ist so gewählt, dass keine Selbsthemmung auftreten kann. Vorzugsweise beträgt sie höchstens 70°, gemessen als der Winkel, den die Längsachse der Injektionsnadel mit der Führung oder einer Parallelprojektion der Führung auf die Längsachse einschließt. Eines aus Führung und Eingriffsglied ist relativ zu dem Sicherungsglied und das andere relativ zu wenigstens einem aus Nadelhalter und Blockiereinrichtung ortsfest. So kann das eine, vorzugsweise das Eingriffsglied, insbesondere unmittelbar an dem Sicherungsglied angeordnet, beispielsweise in einem Stück mit diesem geformt sein, während das andere, vorzugsweise die Führung, insbesondere an dem Nadelhalter angeordnet, vorzugsweise in einem Stück mit diesem geformt sein kann. Der Nadelschutz ist mit dem Sicherungsglied so gekoppelt, dass er bei seiner Bewegung in die Auslöseposition auf das Sicherungsglied eine Kraft ausübt, die in dem Führungseingriff von Eingriffsglied und Führung die Bewegung des Sicherungsglieds in die zweite Position bewirkt.

Das Sicherungsglied kann insbesondere in Ausführungen, in denen die Kurvenführung vorgesehen ist, um die Injektionsnadel drehbar gelagert sein. Die Bewegung des Sicherungsglieds aus der ersten in die zweite Position ist vorzugsweise eine überlagerte Translations- und Rotationsbewegung längs der Injektionsnadel und um die Injektionsnadel.

Der Nadelschutz ist vorteilhafterweise über das Federglied mit dem Sicherungsglied gekoppelt, derart, dass der Nadelschutz bei der Bewegung in die Auslöseposition über das Federglied eine in die proximale Richtung wirkende Kraft auf das Sicherungsglied ausübt, so dass dieses aus der ersten in die zweite Position bewegt wird. Vorzugsweise ist das Federglied zwischen dem Nadelschutz und dem Sicherungsglied vorgespannt oder spannt sich zumindest bei der Bewegung des Nadelschutzes in Richtung auf die Freigabeposition. Besonders bevorzugt stützt es sich unmittelbar an dem Nadelschutz und dem Sicherungsglied ab.

In der zweiten Ausführung ist das Sicherungsglied relativ zu der Injektionsnadel und der Blockiereinrichtung aus einer proximalen Position, der ersten Position, die es in einem Ausgangszustand einnimmt, in die distale Richtung in die zweite Position bewegbar. Die Bewegung in die distale Richtung wird durch den Nadelschutz bewirkt, wenn dieser sich nach Erreichen der Auslöseposition in die Schutzposition bewegt. Der Nadelschutz nimmt das Sicherungsglied in die Schutzposition mit. Um die Mitnahme zu bewirken, weisen der Nadelschutz ein Eingriffselement und das Sicherungsglied ein Eingriffsgegenelement auf, die miteinander in einen Mitnahmeeingriff gelangen, wenn der Nadelschutz in die Auslöseposition oder darüber hinaus in die proximale Richtung bewegt wird. Der Mitnahmeeingriff ist vorzugsweise ein Rasteingriff, in dem das Eingriffselement das Eingriffsgegenelement in Bezug auf die distale Richtung hintergreift.

Mit dem Sicherungsglied wird zwar ein weiteres Element eingeführt, die Form des Nadelschutzes oder der Blockiereinrichtung einerseits und insbesondere des Nadelhalters andererseits kann jedoch vereinfacht werden. Im Falle einer radial geschachtelten Anordnung können die für die Blockierung des Nadelschutzes und den Mitnahmeeingriff erforderlichen Formelemente einfach gestaltet werden. Durch eine Anordnung des Sicherungsglieds radial zwischen dem Nadelschutz und der Injektionsnadel kann der Nadelschutz in radialer Richtung versteift werden.

Das Sicherungsglied kann den Nadelschutz bei der Bewegung in die proximale Richtung in einem Gleitkontakt führen. Die Blockiereinrichtung und das Sicherungsglied können zwischen sich beispielsweise einen Ringspalt bilden, in dem der Nadelschutz sowohl an einer Mantelinnenfläche als auch an einer Mantelaußenfläche je im Gleitkontakt geführt wird. In solch einem Ringspalt kann vorteilhafterweise auch eine mechanische Rückstellfeder für den Nadelschutz angeordnet sein, wobei der Ringspalt vorteilhafterweise auch die Rückstellfeder axial führt. Ein Ringraum kann alternativ auch als gasdichte Ringkammer gebildet sein, deren Volumen durch die Bewegung des Nadelschutzes verringert oder vergrößert wird, um durch einen auf diese Weise erzeugten Über- oder Unterdruck die Elastizitätskraft für die Rückstellung des Nadelschutzes in die distale Richtung zu erzeugen.

In der zweiten Ausführung dient das Eingriffselement des Nadelschutzes vorzugsweise nicht nur der Mitnahme des Sicherungsglieds, sondern in Doppelfunktion auch der Blockierung des Nadelschutzes in der Schutzposition. In derartigen Ausführungen stößt das Eingriffselement in der Schutzposition des Nadelschutzes in die proximale Richtung gegen einen Blockieranschlag der Blockiereinrichtung, d.h. eine Bewegung des Nadelschutzes in die proximale Richtung wird durch Anschlagkontakt des Eingriffselements verhindert. Für eine derartige Blockierung in der Schutzposition ist eine radiale Aussteifung des Nadelschutzes mittels des Sicherungsglieds besonders vorteilhaft, indem das Sicherungsglied dem Eingriffselement eine radiale Stütze bietet, um in der Schutzposition eine Bewegung des Eingriffselements nach radial innen, d.h. auf die Injektionsnadel zu, zu blockieren. Das Eingriffselement kann somit nicht versehentlich oder bewusst aus dem Blockiereingriff mit dem Blockieranschlag bewegt werden.

Das Eingriffselement wird bei der Bewegung des Nadelschutzes in die Auslöseposition vorzugsweise gegen eine rückstellende Elastizitätskraft nach radial außen und durch die Elastizitätskraft in den Mitnahmeeingriff bewegt. In dem Mitnahmeeingriff nimmt es vorzugsweise eine Position ein, die weiter radial außen gelegen ist als die Position, die es vor der Injektion im Ausgangszustand eingenommen hat. Die beiden Positionen sind so gelegen, dass das Eingriffselement in den Ausführungen, in denen es in der Schutzposition die Blockierung des Nadelschutzes bewirkt, an dem genannten Blockieranschlag der Blockiereinrichtung vorbei in den Mitnahmeeingriff bewegt werden kann, nach der Bewegung des Nadelschutzes in die Schutzposition jedoch sicher axial auf Anschlag gegen den Blockieranschlag der Blockiereinrichtung gelangt.

Der Blockieranschlag der Blockiereinrichtung kann gegen eine rückstellende Elastizitätskraft nach radial außen bewegbar sein, insbesondere in der zweiten Ausführung. So kann eine elastisch biegbare Zunge den Blockieranschlag bilden. Vorteilhaft ist es, wenn die Zunge der Blockiereinrichtung in die distale Richtung vorragt und dementsprechend ein freies distales Ende aufweist. Das freie distale Ende bildet vorzugsweise den Blockieranschlag.

Ein steifer Blockieranschlag ist ebenfalls eine bevorzugte Option und kann insbesondere in den ersten Ausführungen der Nadelschutzvorrichtung vorgesehen sein. Ein steifer, nicht nachgiebiger Blockieranschlag verhindert besonders sicher, das der Blockiereingriff von Nadelschutz und Blockiereinrichtung durch Manipulationen gelöst und der Nadelschutz aus der Schutzposition bewegt werden kann. Ein steifer Blockieranschlag kann insbesondere dann verwirklicht werden, wenn der Blockieranschlag nicht mit dem Eingriffselement des Nadelschutzes zusammenwirkt, sondern am Nadelschutz zusätzlich zu dem Eingriffselement wenigstens ein Blockierelement für den Blockiereingriff vorgesehen ist, wie dies bevorzugten Varianten der ersten Ausführungsform entspricht. Das Blockierelement kann insbesondere ein elastischer Schnapper sein. Die Blockiereinrichtung ummantelt das Blockierelement im Blockiereingriff vorteilhafterweise.

Das Sicherungsglied bildet in der zweiten Ausführung vorzugsweise eine Führung für das Eingriffselement des Nadelschutzes. Bei der Bewegung des Nadelschutzes in die proximale Richtung, d.h. in Richtung auf die Freigabeposition, wird das Eingriffselement längs der Führung gegen eine rückstellende Elastizitätskraft bevorzugterweise nach radial außen bewegt, bis es in der Auslöseposition in den Mitnahmeeingriff schnappt. Die Führung weist über ihren axialen Verlauf gesehen eine Neigung zu der Achse proximal-distal auf. Die Neigung sollte kontinuierlich, d.h. stetig differenzierbar sein. Sie kann beispielsweise konstant sein oder in die proximale Richtung abnehmen oder, bevorzugter, in die proximale Richtung zunehmen, mit der stärksten Zunahme vorzugsweise in der zweiten proximalen Hälfte oder im letzten proximalen Drittel ihres Verlaufs. Falls das Eingriffselement als elastisch biegbare Zunge geformt ist, sollte diese Zunge in die proximale Richtung vorragen und dementsprechend ein freies proximales Ende aufweisen.

Bevorzugte Merkmale frühauslösender Nadelschutzvorrichtungen offenbart auch die deutsche Patentanmeldung Nr. 102006041128, die diesbezüglich in Bezug genommen wird.

Vorteilhafte Merkmale werden auch in den Unteransprüchen und deren Kombinationen beschrieben.

Nachfolgend wird die Erfindung anhand eines Ausführungsbeispiels erläutert. An dem Ausführungsbeispiel offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: ein erstes Ausführungsbeispiel einer Nadelschutzvorrichtung,
- Figur 2: die Nadelschutzvorrichtung des ersten Ausführungsbeispiels im zusammengebauten Zustand in einer perspektivischen Sicht,
- Figur 3: die Nadelschutzvorrichtung in einem Ausgangszustand,
- Figur 4: die in dem Ausgangszustand befindliche Nadelschutzvorrichtung in einem anderen Längsschnitt als Figur 3,
- Figur 5: die Nadelschutzvorrichtung mit in einer Auslöseposition befindlichem Nadelschutz,
- Figur 6: die Nadelschutzvorrichtung mit in Freigabeposition befindlichem Nadelschutz,
- Figur 7: die Nadelschutzvorrichtung mit in Schutzposition befindlichem Nadelschutz,
- Figur 8: ein zweites Ausführungsbeispiel einer Nadelschutzvorrichtung,
- Figur 9: eine Blockiereinrichtung des zweiten Ausführungsbeispiels
- Figur 10: einen distalen Nadelschutz des zweiten Ausführungsbeispiels,
- Figur 11: einen Nadelhalter des zweiten Ausführungsbeispiels in perspektivischer Darstellung,
- Figur 12: den Nadelhalter in einem Längsschnitt,
- Figur 13: ein Sicherungsglied des zweiten Ausführungsbeispiels in perspektivischer Darstellung,
- Figur 14: das Sicherungsglied in einer Draufsicht in die distale Richtung,
- Figur 15: einen proximalen Nadelschutz des zweiten Ausführungsbeispiels,
- Figur 16: eine Ablaufsteuerung des zweiten Ausführungsbeispiels in einem Ausgangszustand vor einer Injektion,
- Figur 17: die Ablaufsteuerung in einem Endzustand nach einer Injektion,
- Figur 18: die Nadelschutzvorrichtung des zweiten Ausführungsbeispiels in dem Ausgangszustand,
- Figur 19: die Nadelschutzvorrichtung zu Beginn einer Auslösephase,
- Figur 20: die Nadelschutzvorrichtung am Ende der Auslösephase,
- Figur 21: die Nadelschutzvorrichtung mit dem in der Freigabeposition befindlichen Nadelschutz,
- Figur 22: die Nadelschutzvorrichtung mit dem in der Schutzposition befindlichen Nadelschutz,
- Figur 23 bis 25: eine Nadelschutzvorrichtung eines dritten Ausführungsbeispiels.

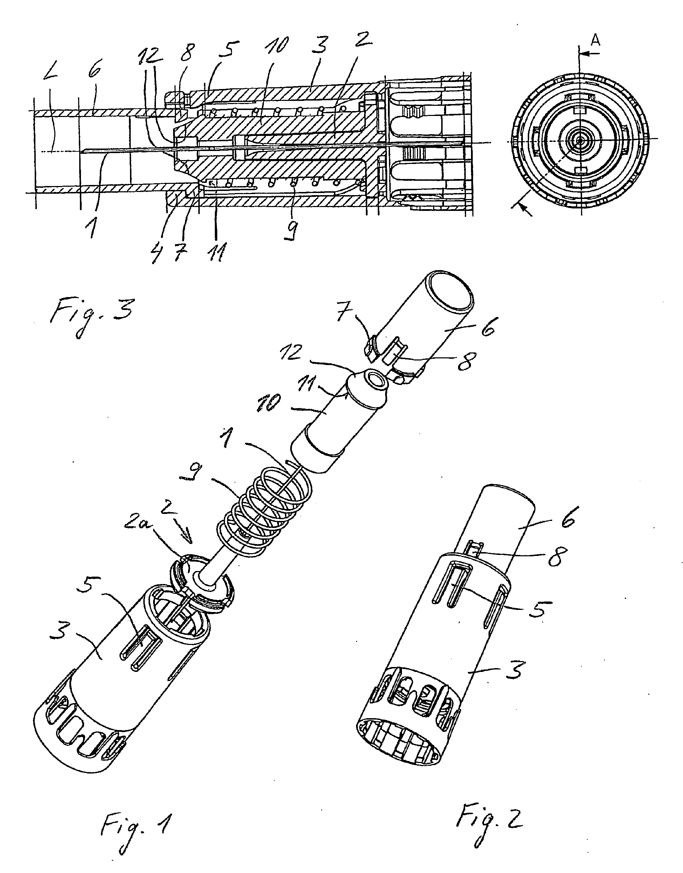

Die Figuren 1 und 2 zeigen in perspektivischer Darstellung eine Nadelschutzvorrichtung, die auf ein distales Ende eines Injektionsgeräts aufsteckbar ist. Bei dem Injektionsgerät handelt es sich vorzugsweise um eines, dass die Einstellung einer Dosis eines injizierbaren Produkts, beispielsweise Insulin, und die wiederholte Abgabe der eingestellten Dosis erlaubt. Vorzugsweise kann die Dosis pro Injektion auch erneut eingestellt werden. Das Injektionsgerät ist bevorzugterweise der Art nach ein Injektionspen, wie er in der Selbstverabreichung von Medikamenten, beispielsweise in der Diabetestherapie, üblich ist. Das Injektionsgerät ist somit für mehrere Injektionen, vorzugsweise für eine längere Gebrauchsdauer, ausgelegt. Die Nadelschutzvorrichtung, die eine Injektionsnadel 1 für das Injektionsgerät umfasst, ist hingegen für nur eine einzige Injektion, d.h. nur für einen einmaligen Gebrauch, bestimmt. Sie wird für den Gebrauch an dem distalen Ende des Injektionsgeräts befestigt, beispielsweise aufgeschraubt oder wie im Ausführungsbeispiel aufgesteckt und nach der Injektion abgenommen und entsorgt.

Die Nadelschutzvorrichtung umfasst wie bereits erwähnt die Injektionsnadel 1, einen Nadelhalter 2 und zwei hülsenförmige Strukturen, nämlich eine äußere Hülse 3 und eine innere Hülse 6, die ein Nadelschutzteleskop bilden. Die innere Hülse 6 ist relativ zu der äußeren Hülse 3 und insbesondere der Injektionsnadel 1 axial beweglich. Figur 2 zeigt die Nadelschutzvorrichtung in einem Ausgangszustand, in dem die innere Hülse 6 relativ zu der äußeren Hülse 3 eine distale Position einnimmt, in der sie die Injektionsnadel 1 bis über deren Nadelspitze verdeckt und so einen Sichtschutz bildet. Die innere Hülse 6 wird im Folgenden deshalb als Nadelschutz 6 bezeichnet. Die äußere Hülse 3 dient der Blockierung des Nadelschutzes 6 nach der Injektion und wird im Folgenden deshalb als Blockiereinrichtung 3 bezeichnet.

Figur 3 zeigt die Nadelschutzvorrichtung ebenfalls in dem Ausgangszustand. Neben dem Längsschnitt ist auch ein Querschnitt dargestellt. In der Querschnittdarstellung ist die Lage des Längsschnitts A-A angegeben.

Die Blockiereinrichtung 3 weist einen proximalen Befestigungsabschnitt für die Befestigung der Nadelschutzvorrichtung an einem Injektionsgerät auf. Der Nadelhalter 2 ist in die hülsenförmige Blockiereinrichtung 3 eingesetzt und im eingesetzten Zustand mit der Blockiereinrichtung 3 axial und um die Längsachse L rotatorisch nicht beweglich verbunden. Soweit es um die Funktion der Blockiereinrichtung 3 in Bezug auf den Schutz vor der Injektionsnadel 1 geht, könnte die Blockiereinrichtung 3 mit dem Nadelhalter 2 in einem Stück geformt sein. Für den Zusammenbau der Nadelschutzvorrichtung ist jedoch eine Fertigung separat von dem Nadelhalter 2 vorteilhaft. Der Nadelhalter 2 weist einen Boden 2a auf, von dem in die distale Richtung im zentralen Bereich ein Halteabschnitt für die Injektionsnadel 1 abragt. Die Längsachse L der Injektionsnadel 1 fällt mit der Achse proximal-distal zusammen. Falls das Injektionsgerät wie bevorzugt das zu injizierende Produkt mittels eines Hubkolbens fördert, fällt die axiale Richtung vorteilhafterweise mit der Vortriebsrichtung des Kolbens zusammen. In dem Halteabschnitt ist die Injektionsnadel 1 axial fest mit dem Nadelhalter 2 verbunden. Die Injektionsnadel 1 durchragt den Halteabschnitt des Nadelhalters 2. Sie ragt in die proximale Richtung in einen von der Wandung umgebenen Raum des Befestigungsabschnitts, steht hinter einem proximalen Rand der Wandung jedoch zurück. Dieser Raum wird von einer Folie, die an dem proximalen Ende an der Wandung des Nadelhalters 2 befestigt ist, steril verschlossen. Bei der Befestigung der Nadelschutzvorrichtung an dem Injektionsgerät wird die Folie zerstört, und die Injektionsnadel dringt mit ihrem proximalen spitzen Ende durch eine Membran, die ein distales Ende eines mit dem Produkt gefüllten Behälters verschließt. Auf diese Weise wird bei der Befestigung an dem Injektionsgerät gleichzeitig auch die Injektionsnadel 1 fluidisch mit dem Produktbehälter verbunden.

Zu ihrem distalen Ende hin weist die Injektionsnadel 1 einen Injektionsabschnitt mit der Nadelspitze auf. Die Länge der Injektionsnadel ist für eine subkutane Injektion bemessen. Entsprechend dringt der Injektionsabschnitt in die Haut und zum Teil durch die Haut bis zu einer subkutanen Injektionsstelle. Der Injektionsabschnitt erstreckt sich von der freien Nadelspitze in die proximale Richtung bis auf die axiale Höhe des distalen Endes der Blockiereinrichtung 3. Der Nadelschutz 6 nimmt in dem Ausgangszustand eine distale Position ein, die durch das Zusammenwirken eines Anschlags 4 der Blockiereinrichtung 3 mit einem Gegenanschlag 7 des Nadelschutzes 6 vorgegeben ist. Den Anschlag 4 bildet eine von der Blockiereinrichtung 3 nach radial innen vorstehende Schulter, und den Gegenanschlag 7 bildet eine von dem Nadelschutz 6 nach radial außen vorstehende Schulter.

Die Nadelschutzvorrichtung umfasst ferner ein Federglied 9, im Ausführungsbeispiel eine mechanische Druckfeder. Das Federglied 9 übt auf den Nadelschutz 6 eine in die distale Richtung wirkende Elastizitätskraft aus, mit der es den Gegenanschlag 7 gegen den Anschlag 4 drückt. Das Federglied 9 ist axial an der Blockiereinrichtung 6 und dem Nadelhalter 2 abgestützt.

Die Nadelschutzvorrichtung umfasst ferner ein Sicherungsglied 10 für den Nadelschutz 6. Das Sicherungsglied 10 verlängert den Halteabschnitt des Nadelhalters 2 in die distale Richtung. Es weist eine zylindrische, im Ausführungsbeispiel kreiszylindrische Mantelaußenfläche auf, die einer zylindrischen, parallel beabstandeten, im Ausführungsbeispiel kreiszylindrischen Mantelinnenfläche der Blockiereinrichtung 3 zugewandt gegenüberliegt. Die Mantelaußenfläche des Sicherungsglieds 10 und die Mantelinnenfläche der Blockiereinrichtung 3 begrenzen radial einen axial erstreckten Ringspalt, in den der Nadelschutz 6 einfahren kann. In dem Ringraum ist auch das Federglied 9 angeordnet. Die den Ringraum zwischen sich begrenzenden Mantelflächen führen den Nadelschutz 6 bei seiner Bewegung in einem Gleitkontakt und führen im Ausführungsbeispiel auch das Federglied 9 bei dessen Ein- und Ausfedern.

Die Mantelaußenfläche des Sicherungsglieds 10 bildet eine Führung 12 für den Nadelschutz 6, im Ausführungsbeispiel eine Gleitführung. Die Führung 12 bildet einen distalen Endabschnitt des Sicherungsglieds 10. Die Führung 12 ist um die Längsachse L umlaufend als glatte Fläche gebildet. Sie erstreckt sich von dem distalen Ende des Sicherungsglieds 10 in die proximale Richtung mit einer Neigung nach radial außen. Das Sicherungsglied 10 verdickt sich an seiner Mantelaußenfläche somit von dem distalen Ende in die proximale Richtung und fällt an einem proximalen Ende der Verdickung bzw. Aufweitung abrupt in einem um die Längsachse L umlaufenden Absatz bis auf einen außen kreiszylindrischen Abschnitt des Sicherungsglieds 10 ab. An diesen im Vergleich mit der Führung 12 axial längeren Abschnitt schließt sich an dem proximalen Ende des Sicherungsglieds 10 ein verdickter Endabschnitt an. Der Übergang zwischen dem mittleren Abschnitt und dem proximalen Endabschnitt ist ebenfalls abrupt in Form eines Absatzes. Die Führung 12 ist von ihrem distalen Ende ausgehend bis zu ihrem proximalen Ende, dem Absatz 11, kontinuierlich gekrümmt. Im Ausführungsbeispiel nimmt die Neigung der Führung 12 von ihrem distalen Ende ausgehend in die proximale Richtung bis nahe zu dem Absatz kontinuierlich zu. Obgleich weniger bevorzugt, könnte die Führung 12 aber auch eine konstante Neigung oder eine in die proximale Richtung degressiv abnehmende Neigung aufweisen.

Der Nadelschutz 6 weist zwei Eingriffselemente 8 auf, die mit der Führung 12 zusammenwirken. Im Schnitt der Figur 3 ist nur eines der Eingriffselemente 8 erkennbar. Das andere Eingriffselement 8 entspricht dem dargestellten der Form nach und auch in seinem Zusammenwirken mit der Führung 12. Das Eingriffselement 8 ist als biegeelastische Zunge geformt, die sich von einem Wurzelbereich im Mantel des Nadelschutzes 6 in die proximale Richtung erstreckt und an ihrem proximalen Ende eine nach radial innen vorspringende Schulter aufweist. Durch die Schulter wird an dem proximalen Ende des Eingriffselements 8 ein nach innen vorragender Nocken erhalten. Dieser Nocken ist mit der Führung 12 in einem Gleitkontakt. Bei einer Bewegung des Nadelschutzes 6 in die proximale Richtung gleitet der Nocken auf der Führung 12, so dass das Eingriffselement 8 aufgrund des geneigten Verlaufs der Führungsbahn 12 allmählich immer stärker elastisch nach außen gebogen wird. Der Absatz am proximalen Ende der Führung 12 bildet ein Eingriffsgegenelement 11 für jedes der Eingriffselemente 8.

Das Sicherungsglied 10 ist mit dem Nadelhalter 2 zwar ausreichend fest verbunden, so dass es die in Figur 2 dargestellte Position nicht aufgrund von Beschleunigungen verlässt, beispielsweise bei Vibrationen oder Stößen, wie sie beim Transport oder der Handhabung üblicherweise auftreten können. Andererseits ist die Verbindung jedoch lösbar. Die Verbindung wird durch Reibschluss zwischen dem Nadelhalter 2, nämlich dessen Halteabschnitt, und dem Sicherungsglied 10 bewirkt. Die reibschlüssig zusammenwirkenden Sitzflächen verjüngen sich konisch in die distale Richtung.

Nach einem Lösen der Reibschlussverbindung ist das Sicherungsglied 10 relativ zu der Injektionsnadel 1 in die distale Richtung gleitbeweglich. Die Injektionsnadel 1 durchragt das Sicherungsglied 10, das hierfür eine zentrale Bohrung aufweist.

Das Sicherungsglied 10 insgesamt weist die Form eines Bolzens auf. Es steift den Nadelschutz 6 und insbesondere dessen Eingriffselemente 8 in Bezug auf die radiale Richtung aus.

Eine wesentliche Funktion der Blockiereinrichtung 3 ist die Herstellung eines Blockiereingriffs mit dem Nadelschutz 6, um diesen in einer distalen Schutzposition gegen eine Bewegung in die proximale Richtung zu blockieren. In der Schutzposition nimmt der Nadelschutz 6 in Bezug auf die Achse L die gleiche Position wie in dem in Figur 3 dargestellten Ausgangszustand ein. Allerdings ist der Nadelschutz 6 in der Schutzposition an einer Bewegung in die proximale Richtung gehindert, nämlich durch die Blockiereinrichtung 3. Zur Erfüllung der Blockierfunktion weist die Blockiereinrichtung 3 zwei Blockierelemente 5 auf. Die Blockierelemente 5 sind je als eine biegeelastische Zunge geformt. Die Blockierelemente 5 ragen in dem Hülsenmantel der Blockiereinrichtung 3 je von einem proximalen Wurzelbereich in die distale Richtung vorsteht. Die Blockierelemente 5 weisen somit je ein freies distales Ende auf. Die distalen Stirnflächen der Blockierelemente 5 bilden Anschlagflächen für die in der Schutzposition des Nadelschutzes 6 axial zugewandt gegenüberliegenden proximalen Stirnflächen der Eingriffselemente 8. Die Blockierelemente 5 bilden so je einen Blockieranschlag und die Eingriffselemente 8 je einen Blockiergegenanschlag für die Blockierung des Nadelschutzes 6 in der Schutzposition. In der Schutzposition umgibt der Nadelschutz den Injektionsabschnitt der Injektionsnadel 1 bis über deren Nadelspitze hinaus.

In dem Ausgangszustand, wie Figur 3 ihn zeigt, sind der Blockieranschlag des Blockierelements 5 und der Blockiergegenanschlag des Eingriffselements 8 außer Eingriff. Das Eingriffselement 8 ragt in die proximale Richtung bis kurz vor die Führung 12 oder liegt zumindest im Wesentlichen spannungsfrei an dem distalen Ende der Führung 12 an. In dieser Position kann es bei dem Einfahren des Nadelschutzes 6 radial innen an dem Blockierelement 5 ungehindert vorbeibewegt werden. Gleiches gilt für das in Umfangsrichtung um 180° versetzt angeordnete Paar aus Blockierelement 5 und Eingriffselement 8. Obgleich die Nadelschutzvorrichtung bevorzugt mehrere Paare der zusammenwirkenden Elemente 5 und 8 umfasst und dies vorteilhafterweise in symmetrischer Anordnung bezüglich der Achse L, genügt zur Blockierung grundsätzlich bereits ein einziges Elementenpaar 5, 8.

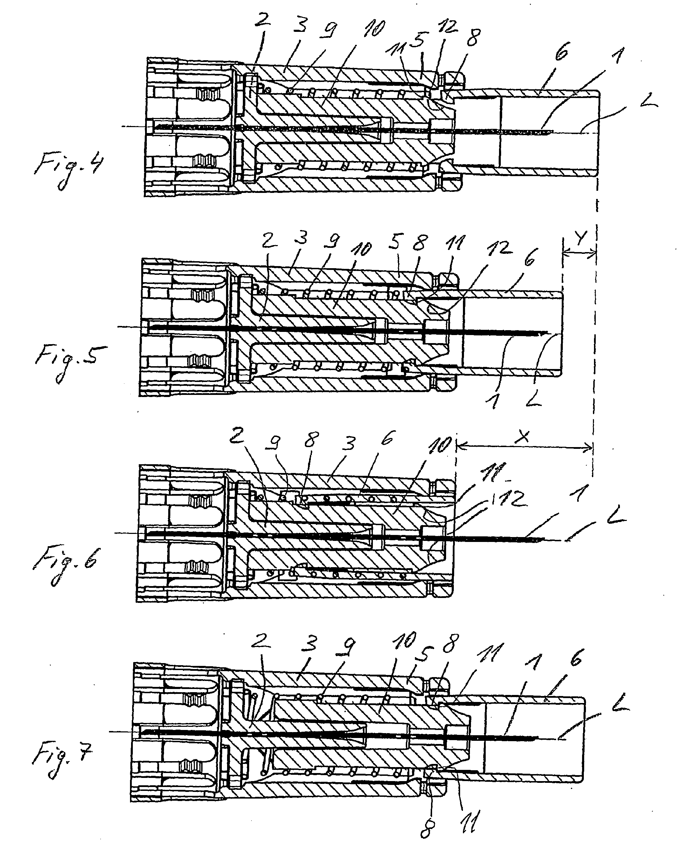

Im Folgenden wird der Ablauf einer Injektion anhand der Figuren 4 bis 7 geschildert:
Nachdem der Verwender die Nadelschutzvorrichtung wie eingangs beschrieben an dem Injektionsgerät befestigt hat, wird die Injektionsnadel 1 entlüftet. Anschließend wählt der Verwender die zu injizierende Dosis. Nach Auswahl der Dosis setzt er das Injektionsgerät mit der distalen Stirnfläche des Nadelschutzes 6 an der gewünschten Einstechstelle auf die Haut und drückt das Injektionsgerät gegen die Haut. Durch die äußere Kraft in die distale Richtung fährt der Nadelschutz 6 axial gegen die Elastizitätskraft des Federglieds 9 in den Ringspalt zwischen der Blockiereinrichtung 3 und dem Sicherungsglied 10 ein. Der Nadelschutz 6 ist relativ zu der Blockiereinrichtung 3 axial verdrehgesichert geführt. Im distal ersten Abschnitt der Bewegung gleiten die Eingriffselemente 8 längs der Führung 12. Die Führung 12 und die Blockierelemente 5 sind so geformt und radial voneinander beabstandet, dass die Eingriffselemente 8 radial innen an dem jeweils zugeordneten Blockierelement 5 vorbeifahren können. Die Blockiereinrichtung 3 weitet sich im Umfangsbereich ihrer Blockierelemente 5 in die proximale Richtung innen soweit auf, dass sich die Eingriffselemente 8 nahezu ungehindert, im Idealfall ohne die Blockierelemente 5 zu verformen, zwischen der Führung 12 und den Blockierelementen 5 nach außen biegen können. Am proximalen Ende der Führung 12 bewegen sich die Eingriffselemente 8 über die dort die Eingriffsgegenelemente 11 distal begrenzenden Kanten und unter ihrer eigenen rückstellenden Elastizitätskraft nach innen; sie schnappen so hinter dem Eingriffsgegenelement 11 ein. Die Eingriffselemente 8 und das Eingriffsgegenelement 11 sind nun miteinander paarweise in einem Mitnahmeeingriff verhakt.

Die Blockierelemente 5 sind an ihrer Führung 12 zugewandten Innenseite wie bereits erwähnt vorzugsweise so geformt, das zwischen der Innenseite und der Führung 12 über den kurzen Anfangshub des Nadelschutzes 6 ein ausreichend großer Abstand verbleibt, damit das jeweilige Eingriffselement 8 keinen oder zumindest keinen das zugeordnete Blockierelement 5 nach außen gerichteten Druck auf das Blockierelement 5 ausübt. Das Eingriffsgegenelement 11 und die Führung 12 sind ferner so geformt, dass das Eingriffselement 8 im Mitnahmeeingriff im Vergleich zu seiner distalen Ausgangsposition nach außen abgebogen ist, im Mitnahmeeingriff also einen größeren Abstand von der zentralen Längsachse L als in der Ausgangsposition aufweist. Im Mitnahmeeingriff weist eine dem jeweiligen Blockierelement 5 zugewandte Außenseite des zugeordneten Eingriffselements 8 von der zentralen Längsachse L einen größeren Abstand auf als das Blockierelement 5 im distalen Bereich an seiner Innenseite.

Figur 4 zeigt die Nadelschutzvorrichtung in einem Ausgangszustand vor Gebrauch, in dem der Nadelschutz 6 die distale Ausgangsposition einnimmt. In der Ausgangsposition wird der Nadelschutz 6 durch die zusammenwirkenden Anschläge 4 und 7 (Figur 3) an der Blockiereinrichtung 3 gehalten. Die beiden Eingriffselemente 8 liegen distal unmittelbar vor der Führung 12 und sind entspannt. Wird auf den Nadelschutz 6 in die proximale Richtung ein axialer Druck ausgeübt, bewegt sich der Nadelschutz 6 relativ zu der Blockiereinrichtung 3 und dem in seiner proximalen Ausgangsposition befindlichen Sicherungsglied 10 in die proximale Richtung. Gleich zu Beginn der Bewegung, nach einem Anfangs- oder Auslösehub Y gelangen die Eingriffselemente 8 in Führungskontakt mit der Führung 12 und biegen sich entsprechend der Neigung der Führung 12 nach radial außen ab. Die Eingriffselemente 8 fahren längs der Führung 12 in den Spalt zwischen dem Sicherungsglied 10 und den Blockierelementen 5, wobei sie stetig stärker nach radial außen gebogen werden, bis das proximale Ende der Führung 12 erreicht ist und die Eingriffselemente 8 aufgrund ihrer elastischen Rückstellkraft hinter das Eingriffsgegenelement 11 nach radial innen schnappen, so dass der Mitnahmeeingriff hergestellt ist. Aus dieser Zwischen- oder Auslöseposition Y kann sich der Nadelschutz nicht mehr in die nicht blockierte Ausgangsposition zurückbewegen, wenn er von der äußeren Druckkraft entlastet und somit sich selbst überlassen wird.

Figur 5 zeigt die Nadelschutzvorrichtung mit dem in der Auslöseposition Y befindlichen Nadelschutz 6. Aufgrund der in die distale Richtung weit vorgerückten Position des Eingriffsgegenelements 11 ist der Mitnahmeeingriff bereits hergestellt, bevor die Spitze der Injektionsnadel 1 in die distale Richtung über den Nadelschutz 6 vorsteht. Im Ausführungsbeispiel steht die Nadelspitze sogar noch ein Stück weit hinter dem Nadelschutz 6 zurück. Weiterer Druck auf den Nadelschutz 6 bewirkt, dass der Nadelschutz 6 gegen die Kraft des Federglieds 9 weiter in die Blockiereinrichtung 3 einfährt. Die Eingriffselemente 8 fahren bei dieser weiteren Einfahrbewegung über die axial gerade, im Ausführungsbeispiel kreiszylindrische Umfangsfläche, die sich in die proximale Richtung an das Eingriffsgegenelement 11 anschließt.

Figur 6 zeigt die Nadelschutzvorrichtung nach Ausführung des Freigabehubs X mit dem in der Freigabeposition befindlichen Nadelschutz 6. Der Nadelschutz 6 ist über seine gesamte Länge in die axiale Überlappung mit der Blockiereinrichtung 3 eingefahren. In diesem voll eingefahrenen Zustand liegen die distalen Stirnenden der Blockiereinrichtung 3, des Nadelschutzes 6 und des Sicherungsglieds 10 auf der gleichen axialen Höhe. Die Injektionsnadel 1 ragt über das gemeinsame Stirnende mit ihrem gesamten Injektionsabschnitt vor, d.h. die Injektionsnadel 1 ist bis in die gewünschte, längs der Injektionsnadel 1 gemessene Eindringtiefe durch die Haut in das darunter liegende subkutane Gewebe eingedrungen, und es kann die eingestellte Dosis verabreicht werden. Während der Verabreichung muss lediglich der für die Überwindung der rückstellenden Elastizitätskraft des Federglieds 9 erforderliche Druck ausgeübt werden. Nachdem die eingestellte Dosis verabreicht wurde, entfernt der Verwender das Injektionsgerät axial in die proximale Richtung von der Einstichstelle, so dass der äußere Druck von dem Nadelschutz 6 genommen wird.

Wird die äußere Kraft von dem Nadelschutz 6 genommen, fährt der Nadelschutz 6 aufgrund der rückstellenden Kraft des Federglieds 9 automatisch wieder in die distale Richtung vor, bis die Eingriffselemente 8 in Anschlagkontakt mit dem Eingriffsgegenelement 11 gelangen. Der Abstand, den das Eingriffsgegenelement 11 in der Ausgangsposition (Figur 4) von der Anlagefläche des Nadelhalters 2 aufweist, ist so bemessen, dass das Sicherungsglied 10 in dem nun wieder erreichten Mitnahmeeingriff der Eingriffselemente 8 und des Eingriffsgegenelements 11 von dem Nadelschutz 6 in die distale Richtung mitgenommen wird. Das gleiche passiert, wenn der Nadelschutz 6 in der Auslöseposition Y von der äußeren Kraft entlastet wird. Im Verlaufe der gemeinsamen Bewegung des Nadelschutzes 6 und des Sicherungsglieds 10 bewegen sich die Eingriffselemente 8 in die distale Richtung vor die Blockierelemente 5 der Blockiereinrichtung 3. Die Eingriffselemente 8, d. h. deren im Eingriff befindliche Enden, befinden sich radial weiter außen als in der Ausgangsposition des Nadelschutzes 6. Bei der Ausfahrbewegung werden die Blockierelemente 5 daher nach radial auswärts abgebogen und schnappen aufgrund ihrer eigenen elastischen Rückstellkraft wieder nach radial einwärts vor, sobald sie von den Eingriffselementen 8 passiert worden sind.

Figur 7 zeigt die Nadelschutzvorrichtung nach der Injektion mit dem in der distalsten Position befindlichen Nadelschutz 6. Am Ende der Bewegung des Nadelschutzes 6 in die distale Richtung verriegelt die Nadelschutzvorrichtung mittels der zusammenwirkenden Eingriffselemente 8 und Blockierelemente 5 automatisch, so dass die distalste Position nun die blockierte Schutzposition des Nadelschutzes 6 ist.

Das Sicherungsglied 10 stützt die Eingriffselemente 8 nach radial innen ab, so dass diese nicht aus dem mit den Blockierelementen 5 gebildeten Blockiereingriff bewegt werden können. Die Blockierelemente 5, Eingriffselemente 8 und Führung 12 sind vorteilhafterweise so geformt, dass die Blockierelemente 5 nur bei der Bewegung des Nadelschutzes 6 in die distale Richtung in einem relevanten Ausmaß elastisch beansprucht werden. Die sich bei dem Einfahren nach außen biegenden Eingriffselemente 8 sollten demgegenüber allenfalls eine deutlich kleinere Biegung der Blockierelemente 5 verursachen. Bevorzugt werden die Blockierelemente 5 beim Einfahren überhaupt nicht belastet. Dies wird im Ausführungsbeispiel durch den im Längsschnitt angepasst geneigten Verlauf der Innenflächen der Blockierelemente 5 und der Führung 12 und die entsprechend schlanken Eingriffselemente 8 erreicht.

Die Eingriffsgegenelemente 11 sind in Bezug auf die axiale Richtung so angeordnet, dass sie in der Schutzposition des Nadelschutzes 6 axial von der Blockiereinrichtung 3 überlappt werden, so dass die Blockiereinrichtung 3 Manipulationen der Eingriffselemente 8, durch die die Eingriffselemente 8 aus dem Mitnahmeeingriff bewegt werden könnten, verhindert.

Der Verwender nimmt nach der Injektion die Nadelschutzvorrichtung von dem Injektionsgerät ab und führt sie der Entsorgung zu. Für eine erneute Injektion wird eine neue, im Ausgangszustand der Figuren 3 und 4 befindliche Nadelschutzvorrichtung an dem Injektionsgerät befestigt.

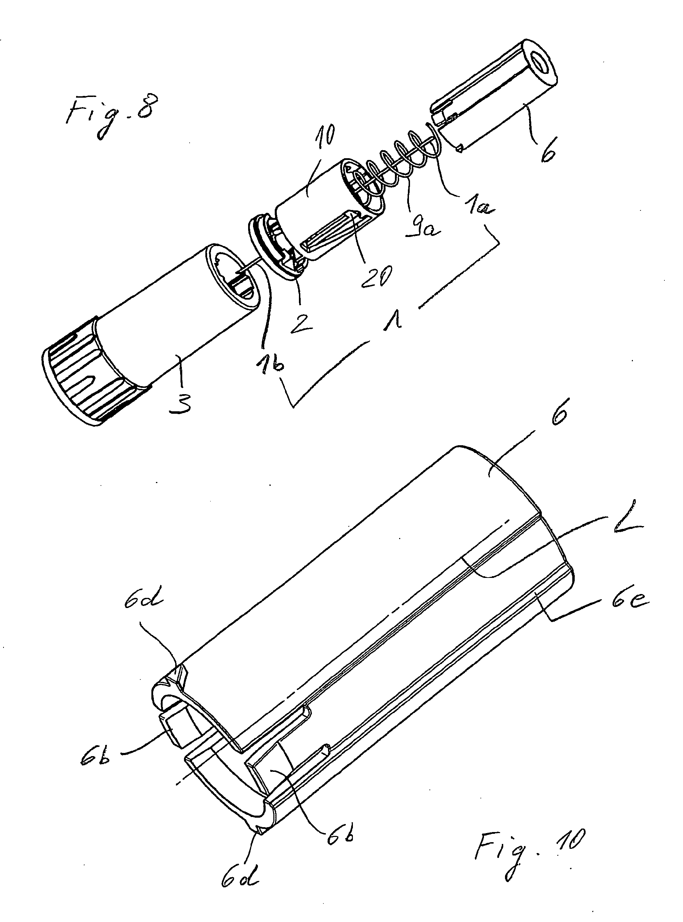

Figur 8 zeigt eine Nadelschutzvorrichtung eines zweiten Ausführungsbeispiels mit ihren längs einer zentralen Längsachse L hintereinander in der Reihenfolge der Montage aufgereihten Komponenten. Die Nadelschutzvorrichtung umfasst eine Injektionsnadel 1, die wie im ersten Ausführungsbeispiel von einem Nadelhalter 2 gehalten wird, eine Blockiereinrichtung 3, die auch wieder eine Befestigungs- und Führungseinrichtung für die Befestigung der Nadelschutzvorrichtung an dem distalen Ende eines Injektionsgeräts und eine axiale, verdrehgesicherte Führung des Nadelschutzes 6 bildet, ein Federglied 9a und ein Sicherungsglied 10. Die Nadelschutzvorrichtung umfasst ferner einen distalen Nadelschutz 6.

Der Nadelhalter 2 ist in der hülsenförmigen Blockiereinrichtung 3 eingesetzt und nicht beweglich befestigt. Ein Nadelinjektionsabschnitt 1a ragt mit einer für subkutane Injektionen geeigneten Länge über die Blockiereinrichtung 3 in die distale Richtung vor. Die Blockiereinrichtung 3 umgibt im proximalen Bereich einen Nadelverbindungsabschnitt 1b und ragt über diesen in die proximale Richtung vor. Der Nadelschutz 6 wird mittels des Sicherungsglieds 10 gegen die Kraft des Federglieds 9a relativ zu der Blockiereinrichtung 3 in der Ausgangsposition gehalten.

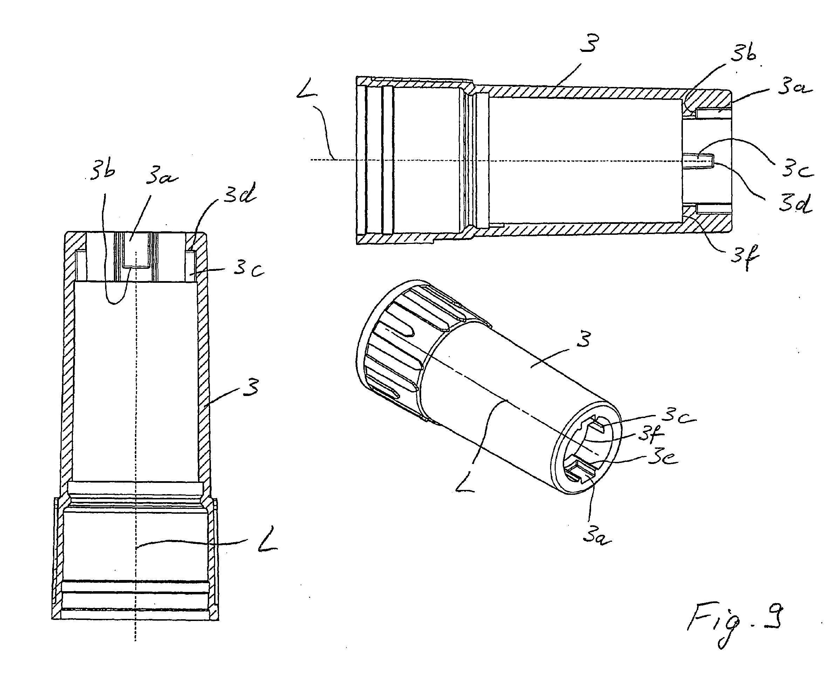

Figur 9 zeigt die Blockiereinrichtung 3 in zwei Längsschnitten, die um 90° zueinander versetzt sind, und einer perspektivischen Sicht. Die Blockiereinrichtung 3 weist am distalen Ende in einer um die Längsachse L umlaufenden Mantelinnenfläche zwei taschenförmige Ausnehmungen 3a auf. Die Ausnehmungen 3a gehen je an ihrem proximalen Ende über eine steile Schulter in die Mantelinnenfläche über. Die beiden Schultern bilden je einen in die distale Richtung weisenden Translationsanschlag 3b. Seitlich von den Ausnehmungen 3a sind jeweils axial erstreckte Führungen 3e geformt für die axiale Geradführung des Nadelschutzes 6. An dem distalen Ende der Blockiereinrichtung 3 sind in der gleichen Mantelinnenfläche zwei weitere taschenförmige Ausnehmungen 3c geformt, die über den Umfang der Mantelinnenfläche zu den Ausnehmungen 3a jeweils um 90° versetzt sind. Die Ausnehmungen 3c gehen je an ihrem distalen Ende über eine steile Schulter in die Mantelinnenfläche über. Die beiden Schultern bilden je einen in die proximale Richtung weisenden Translationsanschlag 3d. Ebenfalls noch im distalen Endabschnitt bildet die Blockiereinrichtung 3 einen Translationsanschlag 3f für das Sicherungsglied 10. Die Translationsanschläge 3b und 3d bestimmen die Schutzposition des Nadelschutzes 6. Der Translationsanschlag 3d dient der Blockierung gegen ein weiteres Ausfahren, und der Translationsanschlag 3b dient der Blockierung gegen ein Einfahren, ersetzt also den Blockieranschlag des Blockierelements 5 des ersten Ausführungsbeispiels.

Figur 10 zeigt den Nadelschutz 6. Der Nadelschutz 6 weist axiale Führungen 6e auf, die mit den Führungen 3e der Blockiereinrichtung 3 zusammenwirken und mit diesen eine verdrehgesicherte Linearführung des Nadelschutzes 6 bilden. Der Nadelschutz 6 weist an seinem proximalen Ende in Umfangsrichtung um 180° zueinander versetzt zwei Blockierelemente 6b auf, die im Ausführungsbeispiel als Federzungen gebildet sind. Die Blockierelemente 6b sind in die proximale Richtung nach außen geneigt. Ferner sind an dem äußeren Umfang des Nadelschutzes 6 ebenfalls an dessen proximalen Ende in Umfangsrichtung um 180° zueinander versetzt zwei Eingriffselemente 6d als nach außen abragende Nocken geformt. Die Eingriffselemente 6d weisen je an einer in Umfangsrichtung um die Längsachse L weisenden Seite eine Neigung zur Längsachse L auf, so dass das jeweilige Eingriffselement 6d an der betreffenden Seite eine Rampe bildet.

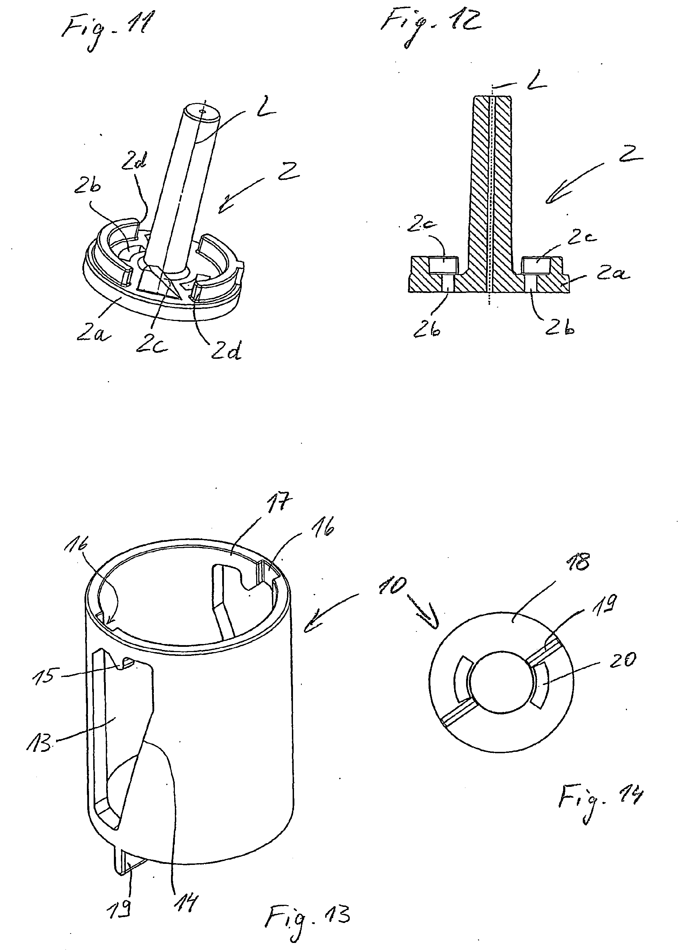

Die Figuren 11 und 12 zeigen den Nadelhalter 2 des zweiten Ausführungsbeispiels. Der Nadelhalter 2 weist einen Boden 2a und einen von dem Boden 2a abragenden zentralen Haltebereich für die Injektionsnadel 1 auf. Der Boden 2a ist mit zwei Durchgängen 2b versehen, durch die der Nadelschutz 21 in die proximale Richtung in eine proximale Schutzposition einfahren kann und die ferner der Blockierung des Nadelschutzes 21 in seiner Schutzposition dienen. Von dem Boden 2a ragen in die distale Richtung außen neben dem Haltebereich zwei in Umfangsrichtung um die Längsachse L um 180° zueinander versetzte Vorsprünge ab, die je an einer distalen Seite rampenförmig geneigt sind, um je eine geneigte Führung 2c für das Sicherungsglied 10 zu bilden. Den rampenförmig geneigten Führungen 2c zugewandt ragt von dem Boden 2a ferner je ein Vorsprung 2d in die distale Richtung ab. Die Ebene des in Figur 12 dargestellten Schnitts erstreckt sich durch die Längsachse L und die Lücken zwischen je einem der die Führungen 2c bildenden Vorsprünge und dem der jeweiligen Führung 2c zugewandten Vorsprung 2d.

Figur 13 zeigt das Sicherungsglied 10 in einer perspektivischen Sicht. Figur 14 zeigt eine Draufsicht auf eine Unterseite des Sicherungsglieds 10, d.h. eine Ansicht in die distale Richtung. Das Sicherungsglied 10 ist hohlzylindrisch. In dem Mantel des Sicherungsglieds 10 sind in Umfangsrichtung um 180° zueinander versetzt zwei Ausnehmungen 13 geformt, die den Mantel durchbrechen und je an einer Seite eine in Umfangsrichtung geneigte Bahn 14 bilden. Von einem distalen Randstreifen des Sicherungsglieds 10 ragt je ein Vorsprung 15 in die proximale Richtung in die jeweilige Ausnehmung 13. Die beiden Ausnehmungen 13 sind von dem Mantel des Sicherungsglieds 10 umlaufend umrahmt, wobei distal vor den Bahnen 14 an der Innenseite des Mantels in Umfangsrichtung neben den Vorsprüngen 15 je ein axial erstreckter Durchgang 16 geformt ist, der sich von dem distalen Ende des Sicherungsglieds 10 durchgehend bis in die jeweilige Ausnehmung 13 erstreckt. Bei der Montage der Nadelschutzvorrichtung wird das Sicherungsglied 10 mit seinen beiden Durchgängen 16 über die Eingriffselemente 6d des Nadelschutzes 6 bewegt, so dass die Eingriffselemente 6d in die jeweils zugeordnete Ausnehmung 13 einfahren. Anschließend werden die Eingriffselemente 6d durch Drehen des Sicherungsglieds 10 ebenfalls noch im Verlaufe der Montage in Umfangsrichtung hinter den jeweiligen Vorsprung 15 in den Umfangsbereich 17 bewegt, so dass die Eingriffselemente 6d den distalen Rand des Sicherungsglieds 10 in dessen Umfangsbereichen 17 hintergreifen. Die beiden Umfangsbereiche 17 bilden je einen Translationsanschlag und in diesem Sinne je ein Eingriffsgegenelement 17 für das jeweils zugeordnete Eingriffselement 6d. Der Nadelschutz 6 wird in diesem Anschlagkontakt gegen die Kraft der Federglieder 9 und 9a an der Blockiereinrichtung 3 gehalten. Die Vorsprünge 15 sind in Querrichtung zu der Längsrichtung der Injektionsnadel 1 jeweils zwischen dem Durchgang 16 und dem Eingriffsgegenelement 17 angeordnet. In einem Führungseingriff mit den Eingriffselementen 6d trennen sie den Durchgang 16 vom jeweils zugeordneten Eingriffsgegenelement 17, so dass die Eingriffselemente 6d entweder in einer ersten Position des Sicherungsglieds 10 in der Überlappung mit den Eingriffsgegenelementen 17 oder in einer zweiten Position des Sicherungsglieds 10 in Überlappung mit den Durchgängen 16 sind. Die Vorsprünge 17 erfüllen die Funktion von Trennelementen.

Das Sicherungsglied 10 ist axial relativ zu dem Nadelhalter 2 und der Blockiereinrichtung 3 hin und her beweglich und ferner um die Längsachse L drehbar.

Das Sicherungsglied 10 weist an seiner Unterseite, am proximalen Ende seines Mantels einen nach radial innen ragenden ringförmigen Boden 18 und von dem Boden 18 in die proximale Richtung abragende Eingriffsglieder 19 auf, insgesamt zwei Eingriffsglieder 19, die um 180° in Umfangsrichtung zueinander versetzt sind. In dem Boden 18 sind zwei um 180° in Umfangsrichtung zueinander versetzte Durchgänge 20 für den proximalen Nadelschutz 21 geformt.

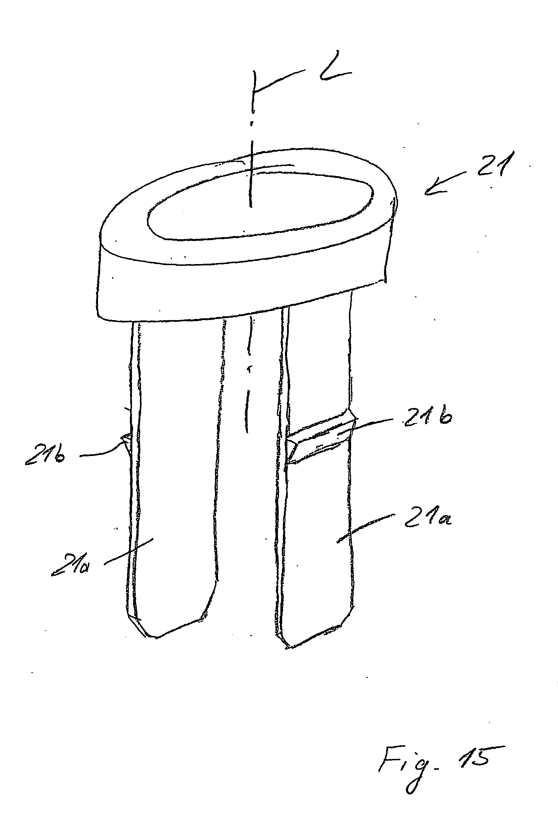

Figur 15 zeigt den proximalen Nadelschutz 21. Der Nadelschutz 21 weist an einem distalen Ende eine ringförmige Basis und von der Basis in die proximale Richtung abragende, elastisch in Richtung auf die Längsachse L biegbare Beine oder Finger 21a auf. An der Außenseite der Finger 21a ist je ein Vorsprung 21b geformt. Die Vorsprünge 21b verjüngen sich pfeilförmig in die proximale Richtung und bilden je einen in die distale Richtung weisenden Anschlag des Nadelschutzes 21.

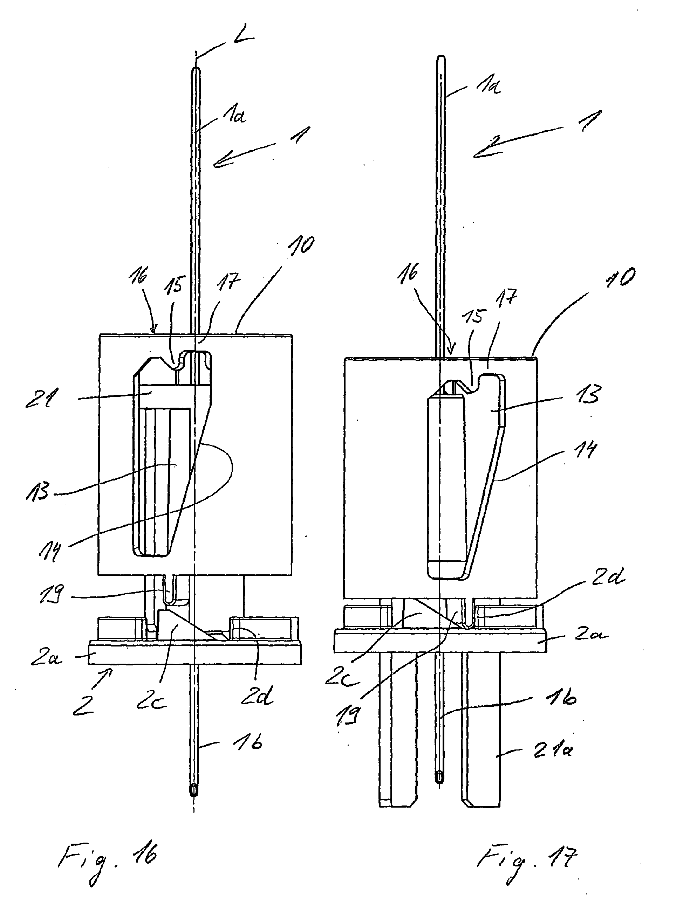

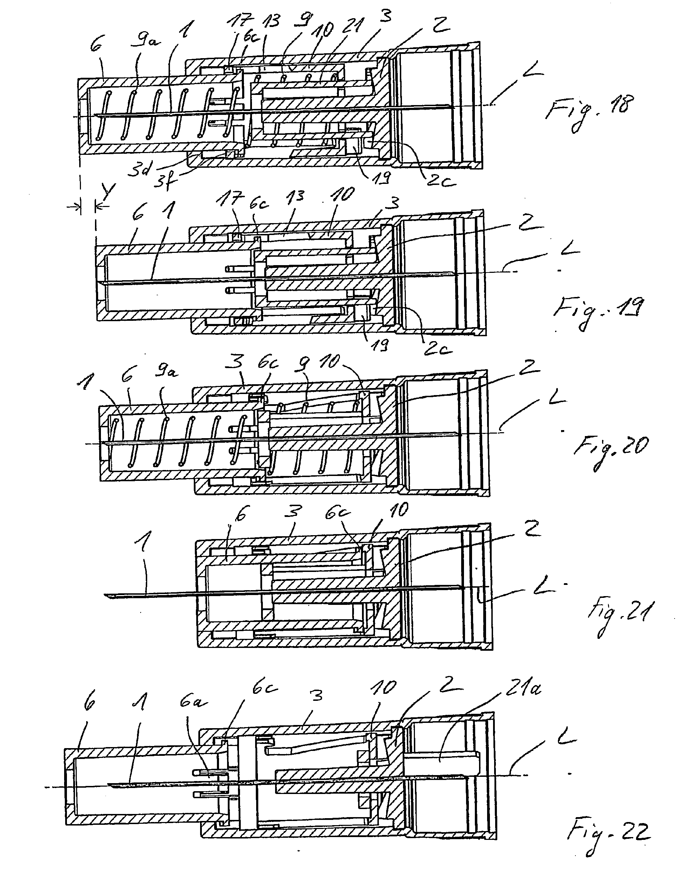

Figur 16 zeigt die bei einer Blockierung des Nadelschutzes 21 zusammenwirkenden Komponenten, wobei der Nadelschutz 21 eine distale Ausgangsposition einnimmt, die gleichzeitig auch einer Freigabeposition des Nadelschutzes 21 entspricht. Der Nadelschutz 21 steht in der Ausgangsposition vollständig hinter dem Verbindungsabschnitt 1b der Injektionsnadel 1 in die distale Richtung zurück. Er steht auch hinter dem Boden 2a des Nadelhalters 2 zurück, d.h. das Federglied 9a drückt den Nadelschutz 21 in dessen Ausgangsposition in die proximale Richtung gegen den Boden 2a. Die Finger 21a durchragen die Durchgänge 20 des Sicherungsglieds 10 (Figur 14). Im Ausgangszustand der Nadelschutzvorrichtung sind die Durchgänge 20 und die Durchgänge 2b des Nadelhalters 2 in Umfangsrichtung zueinander versetzt. Die Eingriffsglieder 19 des Sicherungsglieds 10 sind den Führungen 2c des Nadelhalters 2 axial zugewandt. Das Federglied 9a hält das Sicherungsglied 10 über den Nadelschutz 6 in der in Figur 16 gezeigten distalen Ausgangsposition, indem der Nadelschutz 6 mittels seiner Eingriffselemente 6d das Sicherungsglied 10 in den Umfangsbereichen 17 hintergreift und in die distale Ausgangsposition zieht. Diesen Ausgangszustand zeigt auch Figur 18.

Der Nadelhalter 2 und das Sicherungsglied 10 bilden auch für die Blockierung des Nadelschutzes 6 eine Ablaufsteuerung, um diesen in der Schutzposition zu blockieren, wenn er aus seiner Ausgangsposition zumindest bis in die Auslöseposition bewegt und dann von der die Bewegung in die proximale Richtung bewirkenden äußeren Kraft entlastet wird.

Figur 17 zeigt die Nadelschutzvorrichtung und mit dem in seiner proximalen Schutzposition befindlichen Nadelschutz 21. Figur 22 zeigt die Nadelschutzvorrichtung insgesamt ebenfalls in diesem Endzustand.

Nachfolgend wird die Funktionsweise der Nadelschutzvorrichtung des zweiten Ausführungsbeispiels anhand der Figuren 18 bis 22, aber unter Verweis auf die weiteren Figuren 8 bis 17 und insbesondere auf die Figuren 16 und 17 beschrieben.

Die Nadelschutzvorrichtung wird in dem in Figur 18 dargestellten Ausgangszustand mit dem Injektionsgerät verbunden, beispielsweise verschraubt oder verklippt. Dabei durchdringt der Nadelverbindungsabschnitt 1b die Dichtmembran des Medikamentenreservoirs. Eine Bewegung des Nadelschutzes 21 findet bei der Befestigung noch nicht statt.

Anschließend sticht der Verwender die Injektionsnadel 1 an der gewünschten Einstechstelle durch die Haut in das subkutane Gewebe. Dabei bewegt sich der Nadelschutz 6 relativ zu dem Nadelhalter 2 in die proximale Richtung, so dass umgekehrt der Nadelinjektionsabschnitt 1a vorsticht. Figur 21 zeigt die Nadelschutzvorrichtung im eingestochenen Zustand während der Verabreichung des Medikaments.

Aufgrund der bei dem Einstechen auf den Nadelschutz 6 in die proximale Richtung wirkenden äußeren Kraft bewegt sich der Nadelschutz 6 gegen die rückstellende Federkraft des Federglieds 9a relativ zu dem Nadelhalter 2 und auch relativ zu dem Sicherungsglied 10 in die proximale Richtung. Ein weiteres Federglied 9, das den Nadelschutz 21 umgibt, stützt sich in die proximale Richtung an dem Nadelhalter 2 und in die distale Richtung an dem Nadelschutz 6 ab, im Ausführungsbeispiel an dessen proximalen Ende. Der einfahrende Nadelschutz 6 drückt somit über das Federglied 9 in die proximale Richtung auf das Sicherungsglied 10. Da die Eingriffselemente 6d zu Beginn der Bewegung noch in einem Führungseingriff mit dem jeweils zugeordneten Vorsprung 15 sind und der Nadelschutz 6 verdrehgesichert geradgeführt wird, kann sich das Sicherungsglied 10 noch nicht drehen. Am Ende des Auslösehubs, bei Erreichen der Auslöseposition Y, sind die Eingriffselemente 6d des Nadelschutzes 6 aus der axialen Überlappung mit den Vorsprüngen 15 des Sicherungsglieds 10 bewegt worden. Die axiale Länge der Vorsprünge 15 bestimmt gleichzeitig auch die Länge des Auslösehubs Y, d.h. die auf die Ausgangsposition bezogene Auslöseposition Y. Sobald die Eingriffselemente 6d das proximale Ende des jeweils zugeordneten Vorsprungs 15 passiert haben und dadurch der Führungseingriff der Eingriffselemente 6d und der Vorsprünge 15 aufgehoben ist, können die Eingriffsglieder 19 an den Führungen 2c abgleiten. Das Sicherungsglied 10 dreht sich aufgrund des nunmehr einsetzenden Führungseingriffs der Eingriffsglieder 19 und der Führungen 2c relativ zu dem Nadelhalter 2, der Blockiereinrichtung 3 und auch relativ zu dem Nadelschutz 6 entsprechend der Neigungsrichtung der Führungen 2c um die Längsachse L aus der in Figur 16 dargestellten ersten Position in die Figur 17 dargestellte zweite Position, in der die Eingriffselemente 6d in einer axialen Flucht mit den Durchgängen 16 sind. Die Figuren 19 und 20 zeigen das Sicherungsglied 10 zu Beginn und am Ende seiner Drehbewegung. Unter der Einwirkung der äußeren Kraft fährt der Nadelschutz 6 gegen die Kraft des Federglieds 9 und auch des Federglieds 9a kontinuierlich weiter in Richtung auf die Freigabeposition ein. Die Drehwinkelposition des Sicherungsglieds 10 ändert sich dabei nicht mehr.

Der Drehbewegung ist eine axiale Translationsbewegung überlagert, bei der die Eingriffsglieder 19 des Sicherungsglieds 10 auf der jeweils zugeordneten Führung 2c gleiten. Die Translations- und Rotationsbewegung wird durch Anschlagkontakt der Eingriffsglieder 19 und der Vorsprünge 2d begrenzt. Die Vorsprünge 2d bilden Anschläge für Rotationsbewegung. Das Sicherungsglied 10 nimmt den Nadelschutz 21 bei der Drehbewegung mit, da die Finger 21a die Durchgänge 20 durchgreifen. Sobald das Sicherungsglied 10 seine in Figur 17 dargestellte Neutralposition erreicht hat, überlappen die Durchgänge 20 des Sicherungsglieds 10 (Figur 14) die Durchgänge 2b des Nadelhalters 2 (Figuren 11 und 12), so dass die Finger 21 a des Nadelschutzes 21 unter der Kraft des Federglieds 9a die Durchgänge 2b passieren können. Die Finger 21a werden von dem Federglied 9a bis gegen eine Stelle am distalen Ende des Injektionsgeräts, beispielsweise eine distale Stelle des Geräts selbst oder des Medikamentenreservoirs, gedrückt. Die Finger 21a verhindern nach dem Einfahren in die Durchgänge 2b des Nadelhalters 2, dass sich das Sicherungsglied 10 wieder in seine Ausgangsposition (Figur 16) zurückdrehen kann.

Wenn die Injektionsnadel 1 aus dem Gewebe gezogen und dadurch der Nadelschutz 6 entlastet wird, bewegen die Federglieder 9 und 9a den Nadelschutz 6 in die distale Richtung. Die Position der beiden Vorsprünge 15 ist in Umfangsrichtung so gewählt, dass die Eingriffselemente 6d bei der Bewegung des Nadelschutzes 6 in die distale Richtung in die Durchgänge 16 einfahren, wodurch der Nadelschutz 6 schließlich von dem Sicherungsglied 10 freikommt und die Blockierelemente 6b in die Ausnehmungen 3a der Blockiereinrichtung 3 einfahren können (Figuren 9 und 10) und durch Blockiereingriff mit den Blockieranschlägen 3b den Nadelschutz 6 in seiner distalen Schutzposition gegen eine Bewegung in die proximale Richtung blockieren. Die Eingriffselemente 6d verhindern im Zusammenwirken mit den Anschlägen 3d, dass der Nadelschutz 6 aus der Blockiereinrichtung 3 in die distale Richtung vollständig herausfahren kann.

Die Nadelschutzvorrichtung wird mit dem in seiner Schutzposition blockierten Nadelschutz 6 von dem Injektionsgerät gelöst. Der proximale Nadelschutz 21 fährt unter der Einwirkung des Federglieds 9a in seine in Figur 22 dargestellte Schutzposition in die proximale Richtung relativ zu dem Nadelhalter 2 aus. In der Schutzposition ragen die Finger 21a über die Spitze des Nadelverbindungsabschnitts 1b in die proximale Richtung vor. Der Nadelschutz 21 wird durch einen Blockiereingriff zwischen seinen Vorsprüngen 21b und dem Boden 2a des Nadelhalters 2 gegen eine Bewegung zurück in die distale Richtung blockiert. Der Nadelschutz 21 stützt sich über seine ringförmige Basis in die proximale Richtung an dem Nadelhalter 2 ab.

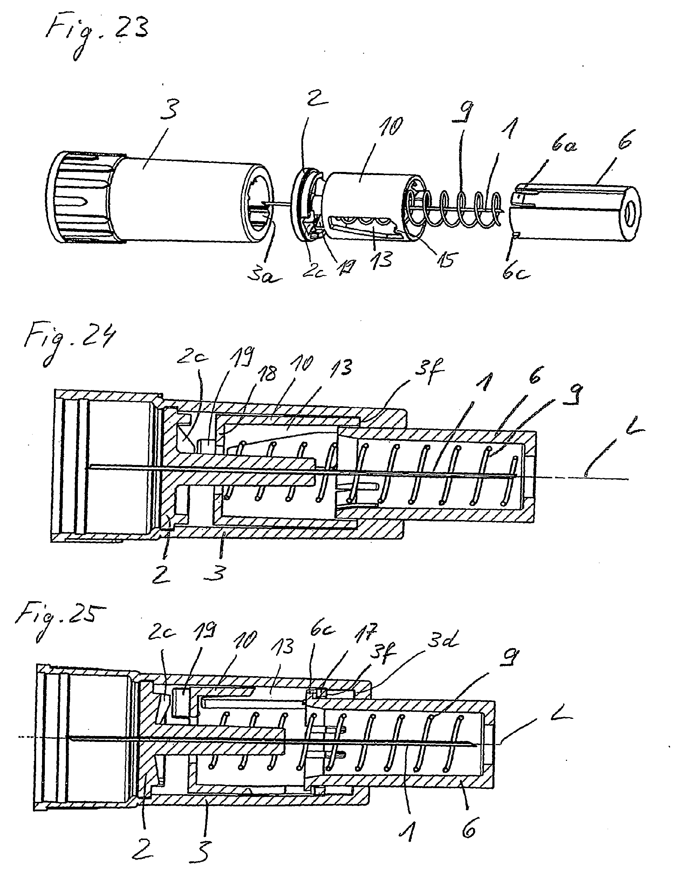

Figur 23 zeigt eine Nadelschutzvorrichtung eines dritten Ausführungsbeispiels. Das dritte Ausführungsbeispiel ist eine Modifikation des zweiten Ausführungsbeispiels. Es wurde auf den proximalen Nadelschutz 21 verzichtet. Dementsprechend ist das Federglied 9a weggefallen. Bei dem Nadelhalter 2 und dem Sicherungsglied 10 sind die Durchgänge für die Finger 21a des Nadelschutzes 21 entfallen. Ansonsten entspricht das dritte Ausführungsbeispiel dem zweiten Ausführungsbeispiel, insbesondere ist dessen Ablaufsteuerung in identischer Form übernommen worden.

Die Figuren 24 und 25 zeigen die Nadelschutzvorrichtung des dritten Ausführungsbeispiels in zwei um 90° zueinander versetzten Längsschnitten. In den Figuren 24 und 25 nimmt der Nadelschutz 6 jeweils seine nicht blockierte Ausgangsposition ein. Das Federglied 9 ist gegenüber dem Federglied 9 des zweiten Ausführungsbeispiels verlängert, indem es sich wie im zweiten Ausführungsbeispiel in die proximale Richtung an dem Boden des Sicherungsglieds 10, aber in die distale Richtung nahe dem distalen Ende des Nadelschutzes 6 an diesem abstützt. Was die für den Bewegungsablauf bei einer Injektion und insbesondere auch die Frühauslösung des Nadelschutzes 6 betreffenden Komponenten und deren Zusammenwirken betrifft, wird auf die Ausführung zum zweiten Ausführungsbeispiel verwiesen.

### Bezugszeichen:

- 1: Injektionsnadel
- 2: Nadelhalter
- 2a: Boden
- 2b: Durchgang
- 2c: Führung
- 2d: Vorsprung
- 3: Blockiereinrichtung
- 3a: Ausnehmung
- 3b: Blockieranschlag, Blockiergegenelement
- 3c: Ausnehmung
- 3d: Anschlag
- 3e: Führung
- 3 f: Anschlag
- 4: Anschlag
- 5: Blockiergegenelement
- 6: Nadelschutz
- 6b: Blockierelement
- 6d: Eingriffselement
- 6e: Führung
- 7: Gegenanschlag
- 8: Eingriffselement
- 9: Federglied
- 9a: Federglied
- 10: Sicherungsglied
- 11: Eingriffsgegenelement
- 12: Führung
- 13: Ausnehmung
- 14: Bahn
- 15: Vorsprung, Trennelement
- 16: Durchgang
- 17: Eingriffsgegenelement
- 18: Boden
- 19: Eingriffsglied
- 20: Durchgang
- 21: proximaler Nadelschutz
- 21 a: Finger
- 21b: Vorsprung
- L: Längsrichtung, Längsachse
- X: Freigabehub, Freigabeposition
- Y: Auslösehub, Auslöseposition

## Patentansprüche

1. Nadelschutzvorrichtung für ein oder an einem Injektionsgerät, die Nadelschutzvorrichtung umfassend:
a) eine injektionsnadel (1),
b) einen an dem injektionsgerät befestigten oder befestigbare Nadelhalter (2), von dem die Injektionsnadel (1) in eine distale Richtung vorragt,
c) ein Federglied (9),
d) einen Nadelschutz (6), der mittels einer äusseren Kraft gegen eine Federkraft des Federglieds (9) aus einer distalen Ausgangsposition, in der er die Injektionsnadel (1) bis über eine Nadelspitze umgibt, in die proximale Richtung um einen Freigabehub (X) bis in eine die Injektionsnadel (1) freigebende Freigabeposition und aus der Freigabeposition in die distale Richtung bis in eine Schutzposition bewegbar ist, in der er die Injektionsnadel (1) ebenfalls bis über die Nadelspitze umgibt,
e) eine Blockiereinrichtung (3), die den Nadelschutz (6) in der Schutzposition in einem Blockiereingriff gegen eine Bewegung in die proximale Richtung blockiert, wobei der Nadelschutz (6) sich aufgrund der Federkraft in den Blockiereingriff bewegt, wenn der Nadelschutz (6) aus der Ausgangsposition um einen kleineren Teil des Freigabehubs (X) bis wenigstens in eine Auslöseposition (Y) bewegt und in der Auslöseposition (Y) von der äusseren Kraft entlastet wird, wobei
ein Sicherungsglied (10) vorgesehen und relativ zu wenigstens einem aus Nadelhalter (2) und Blockiereinrichtung (3) beweglich ist und der Nadelschutz (6) bei Erreichen der Auslöseposition (Y) mit dem Sicherungsglied (10) in einen Eingriff (8, 11; 6d, 16) gelangt, der eine Bewegung des Nadelschutzes (6) in die Schutzposition bewirkt, wenn der Nadelschutz (6) von der äusseren Kraft entlastet wird, oder von einem Eingriff (6d, 17) mit dem Sicherungsglied (10) freikommt, der die Bewegung in die Schutzposition verhindert, wobei
der Nadelschutz (6) ein Eingriffselement (8) und das Sicherungsglied (10) ein Eingriffsgegenelement (11) aufweisen, die miteinander in einen Mitnahmeeingriff gelangen, wenn der Nadelschutz (6) die Auslöseposition (Y) erreicht, und dass der Nadelschutz (6) in dem Mitnahmeeingriff das Sicherungsglied (10) bei der Bewegung in die distale Richtung mitnimmt, **dadurch gekennzeichnet, dass**
f) das Eingriffselement (8) in der Schutzposition des Nadelschutzes (6) in die proximale Richtung gegen einen Blockieranschlag (5) der Blockiereinrichtung (3) stösst.

2. Nadelschutzvorrichtung nach Anspruch 1, **dadurch gekennzeichnet, dass** eine in Längsrichtung (L) der Injektionsnadel (1) erstreckte biegeelastische Zunge das Eingriffselement (8) bildet.

3. Nadelschutzvorrichtung nach Anspruch 2, **dadurch gekennzeichnet, dass** ferner eine weitere Zunge den Blockieranschlag bildet.

4. Nadelvorrichtung nach einem der vorhergehenden Ansprüche, **dadurch gekennzeichnet, dass** das Sicherungsglied (10) das Eingriffselement (8) nach radial innen stützt, um in der Schutzposition eine Bewegung des Eingriffselements (8) nach radial innen zu blockieren.

5. Nadeischutzvorrichtung nach einem der vorhergehenden Ansprüche, **dadurch gekennzeichnet, dass** das Sicherungsglied (10) eine Führung (12) für das Eingriffselement (8) aufweist, die in proximaler Richtung mit einer Neigung nach radial aussen verläuft, um das Eingriffselement (8) bei der Bewegung in die Auslöseposition (Y) nach aussen zu lenken.

6. Nadelschutzvorrichtung nach einem der vorhergehenden Ansprüche, **dadurch gekennzeichnet, dass** die Führung (12) an einem proximalen Ende nach radial innen abfällt, um das Eingriffsgegenelement (11) zu bilden.

## Claims

1. A needle guard device for or on an injection device, the needle guard device including:
a) an injection needle (1),
b) a needle holder (2) which is or can be fastened to the injection device and from which the injection needle (1) projects in a distal direction,
c) a spring member (9),
d) a needle guard (6) which is movable by means of an external force against a spring force of the spring member (9) from a distal starting position in which it surrounds the injection needle (1) to beyond a needle tip in the proximal direction by a liberation stroke distance (X) into a liberation position of liberating the injection needle (1) and out of the liberation position in the distal direction into a guard position in which it also surrounds the injection needle (1) to beyond the needle tip,
e) a blocking device (3) which blocks the needle guard (6) in the guard position in blocking engagement against a movement in the proximal direction, wherein the needle guard (6) moves into the blocking engagement by virtue of the spring force when the needle guard (6) moves out of the starting position by a smaller part of the liberation stroke distance (X) at least into a triggering position (Y) and in the triggering position (Y) is relieved of the external force, wherein
there is provided a securing member (10) movable relative to at least one of the needle holder (2) and the blocking device (3) and the needle guard (6) when reaching the triggering position (Y) comes into an engagement (8, 11; 6d, 16) with the securing member (10), which causes a movement of the needle guard (6) into the guard position when the needle guard (6) is relieved of the external force or is liberated from an engagement (6d, 17) with the securing member (10), which prevents the movement into the guard position, wherein
the needle guard (6) has an engagement element (8) and the securing member (10) has an engagement counterpart element (11) which come into entrainment engagement with each other when the needle guard (6) reaches the triggering position (Y) and the needle guard (6) entrains the securing member (10) in the condition of entrainment engagement in the movement in the distal direction, **characterised in that**
f) the engagement element (8) pushes against a blocking abutment (5) of the blocking device (3) in the guard position of the needle guard (6) in the proximal direction.

2. A needle guard device according to claim 1 **characterised in that** a flexurally elastic tongue which is extended in the longitudinal direction (L) of the injection needle (1) forms the engagement element (8).

3. A needle guard device according to claim 2 **characterised in that** in addition a further tongue forms the blocking abutment.

4. A needle guard device according to one of the preceding claims **characterised in that** the securing member (10) supports the engagement element (8) radially inwardly to block a movement of the engagement element (8) radially inwardly in the guard position.

5. A needle guard device according to one of the preceding claims **characterised in that** the securing member (10) has a guide (12) for the engagement element (8), which guide extends radially outwardly in an inclined position in the proximal direction to deflect the engagement element (8) outwardly in the movement into the triggering position (Y).

6. A needle guard device according to one of the preceding claims **characterised in that** the guide (12) drops away radially inwardly at a proximal end to form the engagement counterpart element (11).

## Revendications

1. Dispositif de protection d'aiguille pour ou sur un appareil d'injection, le dispositif de protection d'aiguille comprenant :
a) une aiguille d'injection (1),
b) un porte-aiguille (2) fixé sur l'appareil d'injection ou susceptible de l'être, depuis lequel l'aiguille d'injection (1) dépasse dans une direction distale,
c) un élément ressort (9),
d) un protège-aiguille (6) qui est déplaçable au moyen d'une force extérieure à l'encontre de la force élastique de l'élément ressort (9) depuis une position de départ distale, dans laquelle il entoure l'aiguille d'injection (1) jusqu'au-delà d'une pointe d'aiguille, dans la direction proximale à raison d'une course de libération (X), jusque dans une position de libération qui dégage l'aiguille d'injection (1), et à partir de la position de libération dans la direction distale jusque dans une position de protection dans laquelle il entoure l'aiguille d'injection (1) également jusqu'au-delà de la pointe d'aiguille,
e) un système de blocage (3), qui bloque le protège-aiguille (6) dans la position de protection en engagement de blocage à l'encontre d'un déplacement dans la direction proximale, tel que le protège-aiguille (6) se déplace en raison de la force élastique pour venir dans l'engagement de blocage quand le protège-aiguille (6) est déplacé depuis la position de départ sur une petite partie de la course de libération (X) jusqu'au moins dans une position de déclenchement (Y) et est déchargé de la force extérieure dans la position de déclenchement (Y), dans lequel il est prévu un organe de sécurité (10), mobile par rapport à au moins un élément parmi le porte-aiguille (2) et le système de blocage (3) et, lorsqu'il atteint la position de déclenchement (Y), le protège-aiguille (6) parvient en engagement (8, 11 ; 6d, 16) avec l'organe de sécurité (10), engagement qui entraîne un déplacement du protège-aiguille (6) jusqu'à la position de protection quand le protège-aiguille (6) est déchargé de la force extérieure, ou bien se dégage d'un engagement (6d, 17) avec l'organe de sécurité (10) qui empêche le déplacement jusque dans la position de protection, dans lequel
le protège-aiguille (6) comprend un élément d'engagement (8) et l'organe de sécurité (10) comprend un élément d'engagement antagoniste (11), qui parviennent l'un avec l'autre dans un engagement d'entraînement quand le protège-aiguille (6) atteint la position de déclenchement (Y), et de sorte que le protège-aiguille (6) qui se trouve en engagement d'entraînement entraîne l'organe de sécurité (10) lors du déplacement dans la direction distale,
**caractérisé en ce que**
f) dans la position de protection du protège-aiguille (6), l'élément d'engagement (8) vient buter dans la direction proximale contre une butée de blocage (5) du système de blocage (3).

2. Dispositif de protection d'aiguille selon la revendication 1, **caractérisé en ce qu'**une languette élastique en flexion qui s'étend dans la direction longitudinale (L) de l'aiguille d'injection (1) forme l'élément d'engagement (8).

3. Dispositif de protection d'aiguille selon la revendication 2, **caractérisé en ce qu'**une autre languette forme en outre la butée de blocage.

4. Dispositif de protection d'aiguille selon l'une des revendications précédentes, **caractérisé en ce que** l'organe de sécurité (10) soutient l'élément d'engagement (8) radialement vers l'intérieur, afin de bloquer, dans la position de protection, un mouvement de l'élément d'engagement (8) radialement vers l'intérieur.

5. Dispositif de protection d'aiguille selon l'une des revendications précédentes, **caractérisé en ce que** l'organe de sécurité (10) comporte un guidage (12) pour l'élément d'engagement (8), le guidage s'étendant en direction proximale et radialement vers l'extérieur avec une inclinaison afin de dévier l'élément d'engagement (8) vers l'extérieur lors du mouvement jusque dans la position de déclenchement (Y).

6. Dispositif de protection d'aiguille selon l'une des revendications précédentes, **caractérisé en ce que** le guidage (12) descend radialement vers l'intérieur à une extrémité proximale, afin de former l'élément d'engagement antagoniste (11).
